# EUROPEAN PATENT SPECIFICATION

(11) **EP 1 022 007 B1**
(45) Date of publication and mention of the grant of the patent: **29.03.2006**
(21) Application number: 00300317.5
(22) Date of filing: 18.01.2000
(51) Int. Cl.: A61F 13/511

(54) **Topsheet for disposable absorbent articles**
Deckschicht für absorbierende Wegwerfartikel
Couche supérieure pour les articles absorbants jetables

(30) Priority: 20.01.1999 JP 1233599
(43) Date of publication of application: 26.07.2000
(73) Proprietor: UNI-CHARM CORPORATION, Shikokuchuo-shi, Ehime-ken (JP)
(72) Inventor: Kashiwagi, Masahiro, c/o Technical Center, Mitoyo-gun, Kagawa-ken 769-1602 (JP)
(74) Representative: Fitchett, Stuart Paul

(56) References cited:
- EP-A- 0 272 683
- WO-A-93/01786
- WO-A-93/11725
- WO-A-97/02133
- WO-A-97/37625
- GB-A- 2 023 067
- US-A- 4 755 413
- US-A- 5 188 625
- US-A- 5 383 870

## Description

This invention relates to an absorbent article for disposal of body fluids such as a sanitary napkin, a disposable diaper or the like.

Japanese Patent Application Disclosure Gazette (Kokai) No. Bei10-272152 discloses an absorbent article for disposal of body fluids, in which a liquid-pervious topsheet comprises a heat-sealable and hydrophobic upper layer sheet having a plurality of liquid-passages extending therethrough and a heat-sealable and hydrophilic lower layer sheet underlying the upper layer. The topsheet protrudes upward to present an arc-shaped cross-section and, in its protuberant region, said upper and lower layer sheets are sealed with each other by a plurality of debossing spots distributed in a pattern. The upper layer sheet may be formed, for example, by a synthetic resin film and said lower layer sheet may be formed, for example, by a fibrous nonwoven fabric. According to a preferred embodiment, the liquid-passage comprises a tapered capillary formed integrally with the liquid-passage and extending downward until a lower end of the capillary comes in contact with the lower layer sheet. Such capillary functions to accelerate absorption of body fluids and to prevent the amount of body fluids once absorbed from flowing back to the exterior of the article.

The known article is certainly advantageous in that its upper surface protruding in are-shape is kept under tension and therefore reliably placed against the wearer's crotch region with good fitting. In addition, the liquid-passages extending downward from the upper layer sheet are not easily deformed because they are supported by the lower layer sheet. However, the article having a good fitting may often be problematic in ventilation or breathability desired to be maintained between the wearer's skin and the article and apt to cause stuffiness and/or eruption.

In view of the problem as has been described above, it is an object of this invention to provide an absorbent article for disposal of body fluids similar to the article in that the liquid-pervious topsheet comprises upper and lower layer sheets but distinguished from the known article in that the ventilation or breathability desired to be maintained between the topsheet and the wearer's skin is drastically improved.

According to this invention, there is provided an absorbent article for disposal of body fluids comprising a liquid-pervious topsheet defining a body facing surface, a liquid-impervious backsheet defining an undergarment facing surface and a liquid-absorbent core disposed between the topsheet and the backsheet, wherein:
the topsheet comprises an upper layer sheet made of a plastic film and a lower layer sheet made of a fibrous assembly; the upper layer sheet is 0.01 - 0.1mm thick and has a plurality of apertures each having a diameter of 0.2 - 6mm and liquid-passages extending around the apertures through the film each having a length of 0.2 - 4mm, a lower end diameter of 0.1 - 5mm and crests and troughs extending in a predetermined direction of the article and alternating in a direction intersecting the predetermined direction at right angles; and the lower layer sheet has a density of 0.01 - 0.1g/cm³ and a basis weight of 10 - 80g/m², and bonded to a bottom surface of the upper layer sheet so that the liquid-passages extend from the film into the fibrous assembly.

According to one embodiment of this invention, the upper layer sheet is of hydrophobic nature and the liquid-passages are treated to become hydrophilic.

According to another embodiment of this invention, the lower layer sheet is a nonwoven fabric made of thermoplastic synthetic fibers.

According to still another embodiment of this invention, the nonwoven fabric is treated to become hydrophilic.

According to further another embodiment of this invention, a bottom surface of the lower layer sheet is bonded to an upper surface of the absorbent core.
Fig. 1 is a perspective view showing a partially cutaway sanitary napkin of this invention; and
Fig. 2 is a fragmentary sectional view taken along a line II-II in Fig. 1.

Details of an absorbent article for disposal of body fluids proposed by this invention will be more fully understood from the description of a sanitary napkin as a specific embodiment of this invention given hereunder with reference to the accompanying drawings.

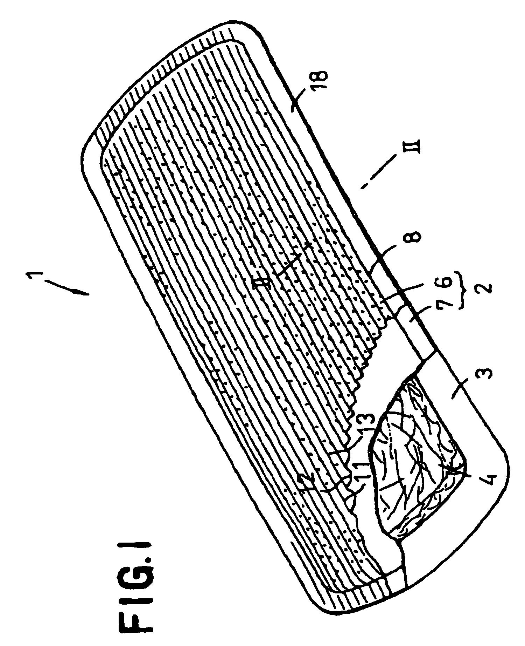

A sanitary napkin 1 shown by Fig. 1 in a perspective view as partially cutaway comprises a liquid-pervious topsheet 2 defining a body facing surface, a liquid-impervious backsheet 3 defining an undergarment facing surface and a liquid-absorbent core 4 disposed between the topsheet and the backsheet 2,3. The topsheet 2 comprises an upper layer sheet 6 and a lower layer sheet 7 bonded to the lower surface of the upper layer sheet 6. The topsheet 2 and the backsheet 3 are put flat together over their portions extending outward beyond a peripheral edge of the absorbent core 4 and bonded to each other along a line 8.

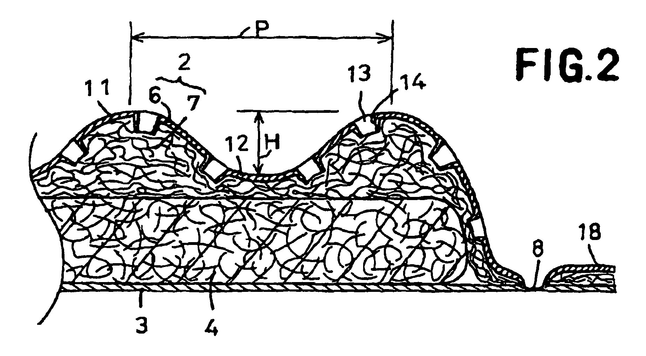

Fig. 2 is a fragmentary sectional view taken along a line II-II in Fig.1 extending transversely of the napkin 1. The upper layer sheet 6 making a part of the topsheet 2 is made of a plastic film having a thickness of 0.01 - 0.1mm and forms a plurality of crests 11 and troughs 12 alternating transversely of the napkin 1 (See Fig. 1 also). A height H of the crest 11 (i.e., a depth of the trough 12) is in a range of 0.3 - 3mm and a pitch between apices of adjacent crests 11 is in a range of 2 - 7mm. The upper layer sheet 6 is formed with a plurality of apertures 13 and liquid-passages 14 extending from the top surface toward the bottom surface around the respective apertures 13. Each of the apertures 13 has a diameter of 0.2 - 6mm and a total area occupied by the apertures 13 corresponds to 20 - 70% of the surface area of the upper layer sheet 6. Each of the liquid-passages 14 has a length of 0.2 - 4mm and a diameter of 0.1 - 5mm at its lower end. The liquid-passages 14 are preferably of hydrophilic nature and if they are formed by a hydrophobic film, peripheral walls of the liquid-passages 14 are preferably treated by suitable means to become hydrophilic.

The lower layer sheet 7 making a part of the topsheet 2 is preferably provided in the form of a fibrous assembly comprising semi-synthetic fibers such as thermoplastic synthetic fibers or rayon fibers or natural fibers such as pulp fibers intertwined or sealed together to have a density of 0.01 - 0.1g/cm³ and a basis weight of 10 - 80g/m² and more preferably provided in the form of nonwoven fabric having a density of 0.01 - 0.1g/cm³ containing 60% or more by weight of thermoplastic synthetic fibers with a fineness of 1 - 8 deniers and a basis weight of 10 - 80g/m². The nonwoven fabric useful for this purpose includes a spun bond nonwoven fabric, a point bond nonwoven fabric, an air-through nonwoven fabric and a spun lace nonwoven fabric. The lower layer sheet 7 is preferably made of hydrophilic fibers or synthetic fibers treated to become hydrophilic. When a certain degree of bulkiness of a cushioning property is desired for the lower layer sheet 7, it is also possible to use a nonwoven fabric containing crimped conjugated fibers. Such lower layer sheet 7 is bonded to the bottom surface of the upper layer sheet 6 by means of adhesive agent or sealing technique and the liquid-passages 14 extend from the upper layer sheet 6 into interfiber gaps of the lower layer sheet 7. Individual fibers of the lower layer sheet 7 surround the respective liquid-passages 14 and serve to protect the liquid-passages 14 from being collapsed. These individual fibers are adhered to or sealed with outer surfaces of the liquid-passage walls as well as their lower openings and thus additionally serve to prevent the liquid-passages 14 from being deformed. While the bottom surface of the lower layer sheet 7 also may have the crests and the troughs similar to those on its upper surface, the lower surface is preferably planar as shown and closely placed against the upper surface of the absorbent core 4. More preferably, the lower layer sheet 7 is intermittently bonded to the upper surface of the absorbent core 4 in a region extending inside the line 8 by means of suitable adhesive agent such as hot melt adhesive agent, sealing technique or embossing in the direction of thickness. Once the upper layer sheet 6, the lower layer sheet 7 and the absorbent core 4 have been bonded together, the crests 11 as well as the troughs 12 can be shifted neither longitudiaally nor transversely of the napkin under a wearer's body weight. Upon removal of the body weight, the crests 11 and troughs 12 immediately restore their initial shapes.

The line 8 along which the topsheet 2 and the backsheet 3 are bonded together is a seal line along which the upper and lower layer sheets 6, 7 and the backsheet 3 are integrally bonded or sealed together and preferably functions to prevent body fluids discharged on the central region of the napkin from exuding toward a peripheral flap 18.

While the napkin 1 has been illustrated and described hereinabove, this invention is applicable also to the other absorbent article for disposal of body fluids such as panty liners, disposable diapers or urine-absorbent pads for incontinent users. It is also possible to use the topsheet 2 according to this invention selectively as a part of the body facing surface of the absorbent article, for example, in the central region of the napkin 1.

The absorbent article for disposal of body fluids such as the napkin arranged as has been described with reference to the accompanying drawings can effectively avoid stuffiness and/or eruption. This is achieved by the body facing surface of the article comprising the crests adapted to be closely placed against the wearer's skin and the troughs adapted to form the ventilating channels. The upper layer sheet forming a part of the topsheet has a plurality of crests and troughs which advantageously makes an effective area of the upper layer sheet substantially larger than that in the case of the planar upper layer sheet. The effective area of the upper layer sheet thus enlarged enables correspondingly increased number of liquid-passages to be present on the surface of the article. In this manner, absorption of body fluids into the absorbent core can accelerated and stuffiness and/or eruption due to use of such article can be further reliably prevented.

The upper layer sheet is backed by the lower layer sheet and therefore the topsheet comprising these two layer sheets has a high elastic restoring force in the direction of thickness particularly in the region defined by the crests. In this manner, the napkin obtains a high cushioning property.

## Claims

1. An absorbent article (1) for disposal of body fluids comprising a liquid-pervious topsheet (2) defining a body facing surface, a liquid-impervious backsheet (3) defining an undergarment facing surface and a liquid-absorbent core (4) disposed between said topsheet and said backsheet, wherein:
said topsheet comprises an upper layer sheet (6) made of plastic film and a lower layer sheet (7) made of fibrous assembly;
said upper layer sheet (6) is 0.01 - 0.1mm thick and has a plurality of apertures (13) each having a diameter of 0.2 - 6mm and liquid-passages (14) extending from said apertures through said upper layer sheet each having a length of 0.2 - 4mm, a lower end diameter of 0.1 - 5mm and crests (11) and troughs (12) extending in a predetermined direction of said article and alternating in a direction intersecting said predetermined direction at right angles; and
said lower layer sheet (7) has a density of 0.01 - 0.1g/cm³ and a basis weight of 10 - 80g/m², and bonded to a bottom surface of said upper layer sheet (6) so that said liquid-passages extend from said upper layer sheet into said lower layer sheet.

2. The article according to Claim 1, wherein said upper layer sheet (6) is of hydrophobic nature and said liquid-passages (14) are treated to become hydrophilic.

3. The article according to Claim 1, wherein said lower layer sheet (7) is a nonwoven fabric made of thermoplastic synthetic fibers.

4. The article according to Claim 3, wherein said nonwoven fabric is treated to become hydrophilic.

5. The article according to Claim 1, wherein a bottom surface of said lower layer sheet (7) is bonded to an upper surface of said absorbent core (4).

## Patentansprüche

1. Absorbierender Artikel (1) zur Beseitigung von Körperflüssigkeiten, der ein flüssigkeitsdurchlässiges Ober-Sheet (2), das eine körperzugewandte Oberfläche definiert, ein flüssigkeitsundurchlässiges Rückseiten-Sheet (3), das eine der Unterwäsche zugewandte Oberfläche definiert und einen flüssigkeitsabsorbierenden Kern (4) aufweist, der zwischen dem Ober-Sheet und Rückseiten-Sheet angeordnet ist, wobei:
das Ober-Sheet ein Oberlagen-Sheet (6) aus einem Kunststofffilm und ein Unterlagen-Sheet (7) aus einem faserigen Aufbau aufweist;
das Oberlagen-Sheet (6) 0,01 - 0,1 mm dick ist und eine Mehrzahl von Öffnungen (13) aufweist, von denen jede einen Durchmesser von 0,2 - 6 mm und Flüssigkeitsdurchlässe (14) hat, die sich von den Öffnungen durch das Oberlagen-Sheet erstrecken, von denen jede eine Länge von 0,2 - 4 mm, einen unteren Enddurchmesser von 0,1 - 5 mm und Gipfelpunkte (11) und Mulden (12) hat, die sich in einer vorgegebenen Richtung des Artikels erstrecken und sich in einer Richtung, die die vorgegebene Richtung unter rechten Winkeln schneidet, abwechseln; und
das Unterlagen-Sheet (7) eine Dichte von 0,01 - 0,1 g/cm³ und ein Basisgewicht von 10 - 80 g/m² hat und auf eine Unterseite des Oberlagen-Sheet (6) aufgeklebt ist, sodass die Flüssigkeitsdurchlässe sich vom Oberlagen-Sheet in das Unterlagen-Sheet erstrecken.

2. Gegenstand gemäß Anspruch 1, wobei das Oberlagen-Sheet (6) eine hydrophobe Beschaffenheit besitzt und die Flüssigkeitsdurchlässe (14) behandelt sind, um hydrophil zu werden.

3. Gegenstand gemäß Anspruch 1, wobei das Unterlagen-Sheet (7) ein ungewebter Stoff aus thermoplastischen Synthetikfasern ist.

4. Gegenstand gemäß Anspruch 3, wobei der ungewebte Stoff behandelt ist, um hydrophil zu werden.

5. Gegenstand gemäß Anspruch 1, wobei eine Unterseite des Unterlagen-Sheet (7) auf eine Oberseite des absorbierenden Kerns (4) geklebt ist.

## Revendications

1. Article absorbant (1) pour l'évacuation de fluides corporels, comprenant une feuille supérieure (2) perméable aux liquides définissant une surface tournée vers le corps, une feuille arrière (3) imperméable aux liquides définissant une surface tournée vers un sous-vêtement, et un noyau (4) absorbant les liquides disposé entre ladite feuille supérieure et ladite feuille arrière, dans lequel :
ladite feuille supérieure comprend une feuille (6) de couche supérieure constituée d'un film de matière plastique et une feuille (7) de couche inférieure constituée d'un ensemble fibreux ;
l'épaisseur de ladite feuille (6) de couche supérieure est de 0,01 à 0,1 mm, et cette feuille comprend une pluralité d'ouvertures (13) d'un diamètre de 0,2 à 6 mm chacune et de passages (14) pour les liquides, dont chacun présente une longueur de 0,2 à 4 mm et un diamètre d'extrémité inférieure de 0,1 à 5 mm, qui s'étendent à partir desdites ouvertures à travers ladite feuille de couche supérieure, ainsi que des crêtes (11) et des creux (12) qui s'étendent dans une direction prédéterminée dudit article et alternent dans une direction qui coupe à angle droit ladite direction prédéterminée ; et
la densité de ladite feuille (7) de couche inférieure est de 0,01 à 0,1 g/cm³ et son poids de base est de 10 à 80 g/m², et elle est attachée à une surface inférieure de ladite feuille (6) de couche supérieure d'une manière telle que lesdits passages pour les liquides s'étendent à partir de ladite feuille de couche supérieure jusqu'à l'intérieur de ladite feuille de couche inférieure.

2. Article selon la revendication 1, dans lequel ladite feuille (6) de couche supérieure est de nature hydrophobe, et lesdits passages (14) pour les liquides sont traités de manière à devenir hydrophiles.

3. Article selon la revendication 1, dans lequel ladite feuille (7) de couche inférieure est une étoffe non tissée de fibres synthétiques thermoplastiques.

4. Article selon la revendication 3, dans lequel l'étoffe non tissée est traitée de façon à devenir hydrophile.

5. Article selon la revendication 1, dans lequel une surface inférieure de ladite feuille (7) de couche inférieure est attachée à une surface supérieure dudit noyau absorbant (4).
